# EUROPEAN PATENT APPLICATION

(11) **EP 3 031 811 A1**
(43) Date of publication of application: **15.06.2016**
(21) Application number: 14196900.6
(22) Date of filing: 09.12.2014
(51) Int. Cl.: C07F 5/02, A61K 38/05, A61P 35/00, A61P 29/00

(54) **Malic acid esters of bortezomib**

(71) Applicant: Teva Pharmaceuticals Ltd., 2100 Gödöllo (HU)
(72) Inventor: Mudri, Denes, 4564 Nyirmada (HU); Vince, Nikolett, 2660 Balassagyarmat (HU)
(74) Representative: Best, Michael

(57) **Abstract**

Disclosed is a new chemical compound which is an ester formed between bortezomib and malic acid, wherein the ester can optionally be in the form of a pharmaceutically acceptable salt. Disclosed are further pharmaceutical compositions, preferably powders for solution for injection and solutions for injection containing the new ester compound or the pharmaceutically acceptable salts thereof as well as processes for preparing said pharmaceutical compositions.

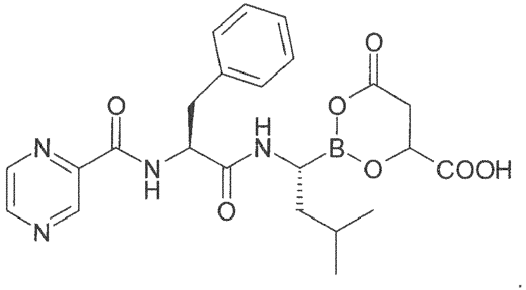

## Description

The present invention relates to a new chemical compound, which is an ester formed between bortezomib and malic acid. The new chemical compound can also be in form of a pharmaceutically acceptable salt of the ester. The present invention also relates to pharmaceutical compositions, preferably powders for solution for injection and solutions for injection, containing the new ester compound or the pharmaceutically acceptable salt thereof, and processes for preparing said pharmaceutical compositions. The present invention also relates to the ester compound, or the pharmaceutically acceptable salt thereof, or the compositions for use in treating cancer.

Peptidyl boronic acids and esters are well-known in the art as inhibitors of certain proteolytic enzymes. Furthermore, they are said to reduce the rate of muscle protein degradation, reduce the activity of NF-κB in a cell, reduce the rate of degradation of p53 protein in a cell, inhibit cyclin degradation in a cell, to mention only some of the physiological activities associated with those compounds.

A problem of many peptidyl boronic acids and esters is an instability to standard conditions of purification and storage. They tend to form anhydrides during dehydration, which can make it difficult to purify the corresponding compounds. They also tend to oxidize in air, which can limit their shelf-life. Furthermore, many peptidyl boronic acids have only a very low solubility in physiological solutions, which makes it difficult to prepare solutions for injection of the compounds.

A particular problem is the low storage stability of these compounds in aqueous compositions which are needed for parenteral administration. In order to solve this problem, it has been suggested to provide a lyophilized powder containing the peptidyl boronic acid, because the stability of the peptidyl boronic acid in the lyophilized powder is much higher than in aqueous compositions. However, a solid composition containing bortezomib, in particular a freeze-dried composition for reconstitution before parenteral administration, should easily and fastly dissolve in the liquid which is intended for the parenteral administration. With many lyophilized products it takes a significant amount of time to completely dissolve the powder before the solution can be parenterally administered.

At present, the peptidyl boronic acid bortezomib 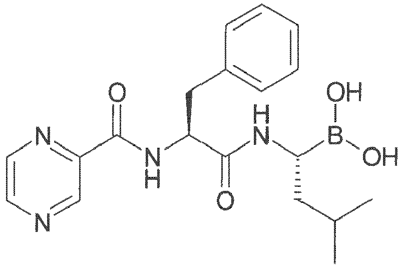 is of particular interest in the pharmaceutical field. This compound in the form of its mannitol ester has received marketing approval for the treatment of multiple myeloma.

WO 02/059130 and WO 02/059131 disclose compounds of a general formula 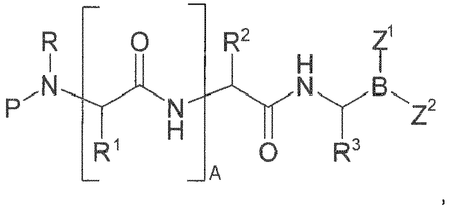 wherein residues Z¹ and Z² are preferably derived from a sugar, wherein the atom attached to boron in each case is an oxygen atom. Thus, the compounds disclosed in those prior art documents are essentially ester compounds formed from a peptidyl boronic acid and at least one sugar.

It is said in the two documents that lyophilization of an aqueous mixture comprising a boronic acid compound and a compound having at least two hydroxyl groups produces a stable composition that readily releases the boronic acid compound upon dissolution in aqueous media.

WO 2009/154737 discloses compounds of a general formula 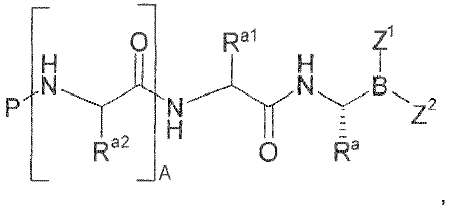 in which Z¹ and Z² together form a moiety derived from an alpha-hydroxy carboxylic acid, wherein the atom attached to boron in each case is an oxygen atom, or Z¹ and Z² together form a moiety derived from a beta-hydroxy carboxylic acid, wherein the atom attached to boron in each case is an oxygen atom. Those ester compounds are said to provide stable pharmaceutically acceptable compositions, and the compounds are said to be especially useful for the treatment of various cell-proliferative diseases.

The document discloses a long list of alpha-hydroxy carboxylic acids and of beta-hydroxy carboxylic acids, including among many others malic acid and citric acid.

WO 2009/154737 also specifically discloses esters of bortezomib with citric acid

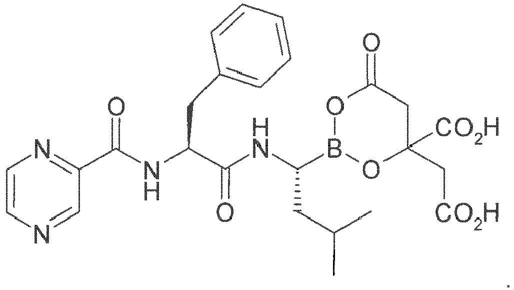

Citric acid esters of the boronic compound are preferred in WO 2009/154737, an example of a malic acid ester compound or the malic acid ester of bortezomib is not disclosed in WO 2009/154737.

WO 2010/089768 discloses that stability problems of parenteral formulations of bortezomib would have been known. Bortezomib shows erratic behavior and is said to be quite unstable in certain solvents. The document provides a parenteral pharmaceutical composition comprising a therapeutically effective amount of bortezomib or its salt or a derivative thereof and tromethamine and an optional bulking agent. The formulation is provided as a lyophilized reconstitutable powder. The bulking agent is selected from the group consisting of potassium chloride, sodium chloride, mannitol, lactose, sucrose, maltose, trehalose and mixtures thereof.

WO 2010/039762 recognizes the need to prepare improved and stable formulations of boronic acid compounds such as bortezomib. Disclosed are sugar-free, lyophilized pharmaceutical products comprising bortezomib or a salt thereof. Several possible stabilizers for the bortezomib including e.g. cyclodextrin and carboxylic acids (among others) are suggested for providing stable bortezomib compositions.

WO 2011/116286 discloses that bortezomib is susceptible to oxidative degradation under a number of experimental conditions and that bortezomib in solution has severe issues with stability. In order to circumvent the stability problems of bortezomib in solutions, the compound can be lyophilized and reconstituted prior to injection. However, this is said to have disadvantages, and pharmaceutical compositions comprising bortezomib in a substantially non-aqueous solvent system suitable for injection are disclosed, wherein the solvent system comprises as a main component propylene glycol.

WO 2012/047845 also discloses that bortezomib, as many other peptidyl boronic acids and esters, is unstable to standard conditions for purification and storage. Several attempts have been made to increase the stability of bortezomib. WO 2012/047845 now provides a composition that includes bortezomib and boric acid. The composition is solid, and the mass ratio of boric acid to bortezomib is from 1:1 to 10:1. The solid composition including bortezomib and boric acid may include other compounds, e.g. an amino acid such as glycine or a saccharide such as dextran or a cyclodextrin or mannitol. In connection with glycine, it is disclosed that the mass ratio of glycine:bortezomib is from 5:1 to 15:1, more preferably from 7:1 to 10:1 or about 7:1.

Despite the many attempts to provide a bortezomib composition for parenteral administration, up to now only one medicament has been approved for medical administration, namely the product VELCADE which is approved for the treatment of multiple myeloma. VELCADE consists of vials containing 1 mg of bortezomib as a mannitol boronic ester in form of a freeze-dried powder. The shelf-life of the unopened vial is said to be good, but after reconstitution the reconstituted solution should be used immediately, and the total storage time for the reconstituted medicinal product should not exceed eight hours prior to administration.

There is a need for further stable compositions of bortezomib which have a long shelf-life and which are suitable for parenteral administration of this compound. The compositions should not only have a long shelf-life, but after reconstitution of a powder, i.e. in form of a solution, the composition should still be sufficiently stable for a sufficient time. Furthermore, reconstitution of a composition in the form of a powder should be fast, preferably less than 10 minutes, more preferably less than 5 minutes, and in particular about 2 to 5 minutes.

It was now found that bortezomib, when it is contacted with malic acid in an aqueous solution, forms an ester bond with the malic acid and that this composition upon freeze-drying has a high storage stability and can easily be reconstituted to form a parenteral solution. It was also found that the presence of glycine in such a composition additionally increases the storage stability of both the freeze-dried composition and the aqueous composition after reconstitution of the freeze-dried composition.

The malic acid ester of bortezomib is a new chemical compound which has not been described before in the prior art.

The present invention therefore provides a new chemical compound which is an ester formed from bortezomib and malic acid or a pharmaceutically acceptable salt of this ester. The present invention also provides a pharmaceutical composition comprising this new bortezomib ester or the salt thereof. Furthermore, the present invention provides a process for preparing such a pharmaceutical composition comprising contacting bortezomib and malic acid in an aqueous medium. The present invention also provides the ester compound or the salt thereof and the pharmaceutical composition for use in treating cancer. Finally, the present invention provides a pharmaceutical composition obtainable by a process which comprises
- mixing bortezomib, malic acid and optionally one or more pharmaceutically acceptable excipients in an aqueous medium or an organic solvent,
- optionally filtering the mixture,
- freeze-drying the mixture, and
- optionally reconstituting the freeze-dried mixture in a pharmaceutically acceptable medium.

The pharmaceutical composition can be in the form of a freeze-dried powder which is easily reconstitutable in a suitable solvent for parenteral administration.

In the present specification, the terms "freeze-drying" and "lyophilization" will be used interchangeably. They have the same meaning. Thus, a freeze-dried product is the same as a lyophilized product.

The present inventors found that the stability of bortezomib is greatly enhanced, if the bortezomib is in the form of an ester. Thus, for the stability of bortezomib compositions it is important that the boron atom is esterified. However, not all bortezomib esters are equally stable, and not all esters of bortezomib can easily be reconstituted in a medium for parenteral administration after lyophilization. The present inventors now found that an ester of bortezomib with malic acid is particularly advantageous when it comes to stability of a freeze-dried composition, reconstitution of the freeze-dried composition, stability of the reconstituted solution and ease of preparation of a pharmaceutical composition.

The malic acid ester of bortezomib according to the invention has the following structure

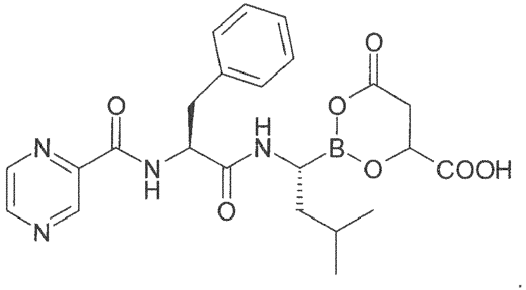

It is theoretically also possible that two malic acid molecules form an ester bond with the boron atom, but such a structure is sterically not favored.

The ester is formed when bortezomib and malic acid are contacted in solution, preferably in an aqueous medium or in an organic solvent. The presence of the ester in an aqueous or organic solution has been confirmed by NMR.

Malic acid is a racemate of the following structure:

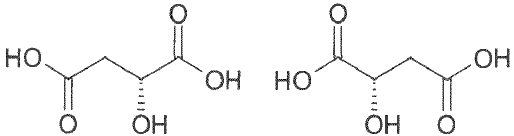

According to the invention, it is possible to use the racemate of malic acid or to use each enantiomer, the L-enantiomer or the D-enantiomer individually or any mixture of the two enantiomers, for forming the ester with bortezomib. Preferably, however, the L-enantiomer is used according to the invention, and therefore, the ester of bortezomib with the L-enantiomer of malic acid is preferred.

The malic acid ester of bortezomib can be in the form as shown above, however, depending on the pH value of the solution and the excipients used for the preparation of the ester, the malic acid ester of bortezomib can also be in the form of a salt, in which e.g. the COOH group and/or one or more of the nitrogen atoms is in salt form. All those forms are encompassed by the term "malic acid ester of bortezomib or a salt thereof" as used in the present specification. In particular, it is possible that the COOH group of the compound of the present invention is deprotonated and/or that one or more of the nitrogen atoms of the compound of the present invention are protonated. If the COOH group of the compound of the present invention is deprotonated, the compound of the present invention may contain a pharmaceutically acceptable counterion for the corresponding anion such as an alkali metal ion, an ammonium ion, or two deprotonated esters of the present invention may share e.g. an earth alkali metal ion. However, the counterions are not specifically restricted, as long as they are pharmaceutically acceptable.

If one or more of the nitrogen atoms of the ester of the present invention is protonated, the counterion can be any pharmaceutically acceptable anion such as a chloride ion or the ion of a pharmaceutically acceptable organic acid. Again, the type of the anion which can be present is not particularly restricted, as long as it is pharmaceutically acceptable.

The malic acid ester of bortezomib is formed in situ when bortezomib and malic acid are contacted in an aqueous medium or organic solvent. According to the invention, it was found that it is not necessary to heat the aqueous or organic composition containing the malic acid and the bortezomib in order to form the malic acid ester of bortezomib. Rather, the malic acid ester of bortezomib forms in situ even at room temperature. However, it is, of course, possible to heat the composition, but this is not preferred.

The ester formation is an equilibrium reaction, and in order to shift the equilibrium to the product side, it is preferred to combine bortezomib with an excess of malic acid in an aqueous medium or an organic solvent. Preferably, in the process for preparing the malic acid ester of bortezomib according to the invention, one mole of bortezomib is contacted in an aqueous medium or an organic solvent with 10 to 25 moles, more preferably with 15 to 25 mole of malic acid, such as 20 moles, 21 moles or 22 mole of malic acid.

From such an aqueous or organic composition containing the malic acid ester of bortezomib, the compound can be isolated by usual purification and isolation methods. In particular, if the malic acid ester of bortezomib is to be isolated, it is preferred to combine malic acid and bortezomib in an aqueous composition or an organic solvent as disclosed above, optionally heating the composition to a temperature not exceeding 50°C, preferably not exceeding 40°C, still more preferably not exceeding 30°C, until the malic acid ester of bortezomib has been formed and then to evaporate the solvent, e.g. by vacuum evaporation. The formation of the ester according to the invention in the composition can be followed by NMR.

Suitable organic solvents for contacting bortezomib and malic acid are alcohols such as isopropyl alcohol, n-propanol, ethanol, tert-butyl alcohol, ethyl acetate, or acetonitrile Particularly preferred as organic solvents are 2-propanol and acetonitrile, and acetonitrile is especially preferred.

The aqueous composition in which the malic acid ester of bortezomib is formed from bortezomib and malic acid can be only water but preferably also contains an organic solvent as disclosed above, most preferably acetonitrile. It has been found that the presence of an organic solvent, in particular of acetonitrile, in the aqueous composition significantly increases the solubility of bortezomib in the aqueous composition and the stability of the bortezomib ester when in the aqueous composition, which in turn facilitates the further handling of the aqueous composition and the isolation of the malic ester of bortezomib.

The organic solvent is preferably present in the aqueous composition in an amount of 100 to 360 mg/ml, preferably of 150 to 360 mg/ml, and more preferably in an amount of 200 to 250 mg/ml.

The aqueous composition is adjusted with suitable acids and/or bases to a suitable pH value which is preferably in the range of 4 to 7, most preferably about 5. Suitable bases and acids for adjusting the pH are sodium hydroxide/hydrochloric acid or potassium hydroxide/hydrochloric acid. Of course, other base/acid pairs can also be used for adjusting the pH value.

Since the malic acid ester of bortezomib of the invention is for use as a parenteral solution or as a powder for reconstitution for a parenteral solution, it is preferred not to isolate the malic acid ester of bortezomib from the aqueous composition or the organic solvent prepared as disclosed above but to directly prepare a pharmaceutical composition from such a composition.

A pharmaceutical composition according to the present invention is preferably prepared by providing an aqueous or organic composition containing bortezomib and malic acid, preferably an excess of malic acid, as described above and freeze-drying this composition. Usually, the composition is filtered prior to freeze-drying. The freeze-dried product will then contain the malic acid ester of bortezomib and, in a preferred embodiment in which an excess of malic acid has been used, also additional free malic acid. It has been found that the free malic acid in a composition additionally stabilizes the malic acid ester of bortezomib, and therefore, the freeze-dried product containing in addition to the malic acid ester of bortezomib also additional malic acid is particularly stable. The preferred relative amounts of malic ester of bortezomib to additional malic acid is, of course, the same as indicated above in connection with the process for preparing the malic ester of bortezomib by contacting bortezomib and malic acid in aqueous or organic solvent.

The compositions in which the malic acid esters of bortezomib have been prepared generally also contain some amount of free bortezomib, since the reaction of bortezomib with malic acid is an equilibrium reaction. It goes without saying that those compositions containing free bortezomib in addition to the malic acid ester of bortezomib are also encompassed by the present invention. In a preferred embodiment, those compositions contain 30 to 70% of free bortezomib, more preferably 40 to 60%, such as about 50% free bortezomib. However, depending on the reaction conditions and the amount of malic acid used compositions with less than 40%, preferably less than 20%, more preferably less than 5% and most preferably less than 1% of bortezomib of the malic acid ester can also be obtained.

In principle, the freeze-dried pharmaceutical composition of the present invention does not need to contain any other components except the malic acid ester of bortezomib (and free bortezomib) and, preferably, additional malic acid (the solvents and co-solvents of the aqueous composition used for preparing the malic acid ester of bortezomib are removed during the freeze-drying process) and residues from the optional acids/bases which have been used for adjusting the pH value.

It is preferred that the freeze-dried composition also contains a bulking agent, and this bulking agent is added to the composition prior to freeze-drying. In principle, every known bulking agent suitable for freeze-drying can be used, but it should be considered that bulking agents which form esters or ethers or otherwise react with bortezomib under the conditions used for preparing the pharmaceutical compositions of the present invention should be avoided in order to prevent the formation of other esters or of ethers of bortezomib in addition to the malic acid ester according to the invention. Thus, the pharmaceutical compositions of the present invention preferably do not contain saccharides or hydroxycarboxylic acids such as citric acid. Preferably, the only hydroxycarboxylic acid present in the pharmaceutical compositions of the present invention is malic acid.

A particularly preferred bulking agent for use in the pharmaceutical compositions of the present invention is glycine. It was found that the presence of glycine as a bulking agent further stabilizes the pharmaceutical compositions of the present invention. In particular, the presence of glycine increases the stability of a solution containing the malic acid ester of bortezomib according to the invention. The presence of glycine is therefore particularly advantageous in preparing a composition for freeze-drying but also after the freeze-dried product has been reconstituted. The reconstituted pharmaceutical compositions with glycine have a higher stability than the corresponding solutions not containing glycine. After reconstitution, the solutions with glycine can be used for a longer period of time than the solutions not containing glycine. Glycine is also advantageous for the stability of the freeze-dried product.

The relative amount of glycine to the malic acid ester of bortezomib in the pharmaceutical compositions of the present invention is not particularly restricted. However, it is preferred that the pharmaceutical compositions of the present invention contain an excess of malic acid compared to bortezomib. On a weight basis the amount of glycine to bortezomib is in a range of preferably 3:1 to 6:1, more preferably in a range of 4:1 to 5:1. The above values are calculated on the basis of the weight of bortezomib used for preparing the composition to be freeze dried and not based on the weight of the malic acid ester of bortezomib (for practical reasons, because usually the malic acid ester of bortezomib is not isolated from the composition used for preparing the pharmaceutical composition of the present invention).

The presence of additional stabilizers for bortezomib disclosed in the prior art is not necessary in the pharmaceutical compositions of the present invention, because the malic acid ester of bortezomib (and free bortezomib in the composition), optionally in the presence of additional malic acid and optionally in the presence of glycine, has been shown to be sufficiently stable for the required purposes. Therefore, preferably the pharmaceutical compositions of the present invention do not contain tromethamine, cyclodextrin or boric acid, which have been used in the prior art for stabilizing bortezomib and bortezomib compounds. It is also not necessary that the pharmaceutical compositions of the present invention contain antioxidants in order to prevent oxidation of bortezomib.

Most preferably, the pharmaceutical compositions of the present invention contain only the malic acid ester of bortezomib (and free bortezomib), malic acid, glycine and any residues which might be present from the compounds used for adjusting the pH value. The reconstituted solutions of the present invention additionally, of course, comprise the medium, usually water (which may contain usual additives), used to prepare the pharmaceutical composition of the present invention for parenteral administration.

It is well-known that parenteral compositions must have a suitable osmolality in order to be safe for administration. A generally accepted reasonably safe range for the osmolality is 150 to 600 mOsmol/kg. The osmolality depends on the concentration of osmotically active compounds in a solution and can be adjusted e.g. by adjusting the amount of malic acid and base (such as sodium hydroxide) used for adjusting the pH value. The osmolality of a pharmaceutical composition of the present invention which is a solution for injection can e.g. be in the range of 300 to 600 mOsmol/kg, preferably from 400 to 600 mOsmol/kg.

The freeze-dried pharmaceutical composition can be packed in vials for storage as is known in the art e.g. from the product VELCADE. As with the product VELCADE, preferably vials are provided containing 1 mg bortezomib or 3.5 mg bortezomib (as a mixture of free bortezomib and its malic acid boronic ester). Preferred are also vials containing 2 mg, 2.5 mg, 3 mg, 4 mg or 5 mg, more preferred 2 mg or 3 mg or 2.5 mg of bortezomib (as a mixture of free bortezomib and its malic acid boronic ester). For reconstitution, an amount of a solvent, in particular aqueous solvent, is used to provide a solution of e.g. 1 mg, 2 mg, 2.5 mg, 3 mg, 3.5 mg, 4 mg or 5 mg, preferably 1 mg, 2.5 mg or 3.5 mg of bortezomib (as a mixture of free bortezomib and its malic ester) per ml of the solution. However, other concentrations of bortezomib are also possible. Any suitable vehicle for providing solutions for injection can be used, and again it can be referred to the recommendations provided for the product VELCADE. Thus, reconstitution is usually done in an aqueous medium containing water for injection and optionally sodium chloride as is known in the art. The reconstituted solution has a pH value of preferably 4 to 7.

Since it is known that bortezomib is subject to oxidation, it is preferred that during the complete production process for the malic acid ester of bortezomib and for the pharmaceutical compositions containing the malic acid ester of bortezomib, an inert gas atmosphere, such as a nitrogen gas atmosphere, is provided. When the freeze-dried product is stored in vials, the vials also preferably contain nitrogen as protective gas.

Pharmaceutical compositions are preferably prepared by lyophilization (freeze-drying) of aqueous compositions having the following constituents

| Component | Parts by weight | Parts by weight | Parts by weight |
|---|---|---|---|
| Bortezomib | 1 | 1 | 1 |
| Malic acid | 3 to 9 | 5 to 8 | 7.35 |
| Glycine | 3 to 7 | 4 to 6 | 5.0 |
| Sodium hydroxide (solid) | 2 to 5 | 3 to 4 | 3.5 |
| Sodium hydroxide (solution) | q.s. pH = 4.9-5.1 | q.s. pH = 4.9-5.1 | q.s. pH = 4.9-5.1 |
| Aqueous medium | q.s. 1000 parts by volume | q.s. 1000 parts by volume | q.s. 1000 parts by volume |

In the above compositions, the aqueous composition preferably consists of water for injection and acetonitrile. The amount of acetonitrile is as indicated above, and therefore, in each of the compositions above, the amount of acetonitrile is 100 to 360 parts, preferably 200 to 250 parts and most preferably 235.8 parts (by weight).

The above compositions are then freeze-dried in order to provide the preferred freeze-dried pharmaceutical compositions of the present invention. The freeze-dried compositions can be reconstituted in the solvent for parenteral administration, and the solvent for parenteral administration preferably consist of water for injection, optionally containing a suitable amount of sodium chloride.

The compositions in the table above have the advantage that the lyophilized powders are suitable to be reconstituted to a wide concentration range of bortezomib per ml of solution for injection without increasing the osmolality too much. For example, the above composition with 7.35 parts by weight of malic acid can be reconstituted to a concentration of 1 mg bortezomib per ml solution but also to a concentration of 3.5 mg bortezomib per 1.4 ml solution and even the 3.5 mg/ml solution has an osmolality of only about 475 mOsmol/kg.

If only reconstitution to lower concentrations such as 1 mg bortezomib per ml or 2 mg bortezomib per ml is intended, higher amounts of malic acid can be used and e.g. the following compositions can be used to prepare the pharmaceutical compositions by lyophilization.

| Component | Parts by weight | Parts by weight | Parts by weight |
|---|---|---|---|
| Bortezomib | 1 | 1 | 1 |
| Malic acid | 9 to 18 | 10 to 16 | 14.7 |
| Glycine | 3 to 7 | 4 to 6 | 5.0 |
| Sodium hydroxide (solid) | 5 to 10 | 6 to 8 | 7 |
| Sodium hydroxide (solution) | q.s. pH = 4.9-5.1 | q.s. pH = 4.9-5.1 | q.s. pH = 4.9-5.1 |
| Aqueous medium | q.s. 1000 parts by volume | q.s. 1000 parts by volume | q.s. 1000 parts by volume |

Again, the aqueous medium contains preferably acetonitrile in an amount as indicated above.

The present invention is further described by way of the following non-limiting examples.

### Example 1

### Preparation of a malic acid ester of bortezomib

A vessel was charged with 700 ml water for injection and 300 ml of acetonitrile, 7.35 g of malic acid was added, and it was stirred until the malic acid was completely dissolved. To this solution 1 g of bortezomib (polymorph form I prepared according to WO 2008/075376) was added, and again the mixture was stirred until a solution was obtained. To this solution 3.5 g of solid sodium hydroxide was added, and it was stirred until the sodium hydroxide was completely dissolved. The pH was adjusted to the target pH of 5.0 with one normal sodium hydroxide solution and one normal hydrochloric acid solution, as required.

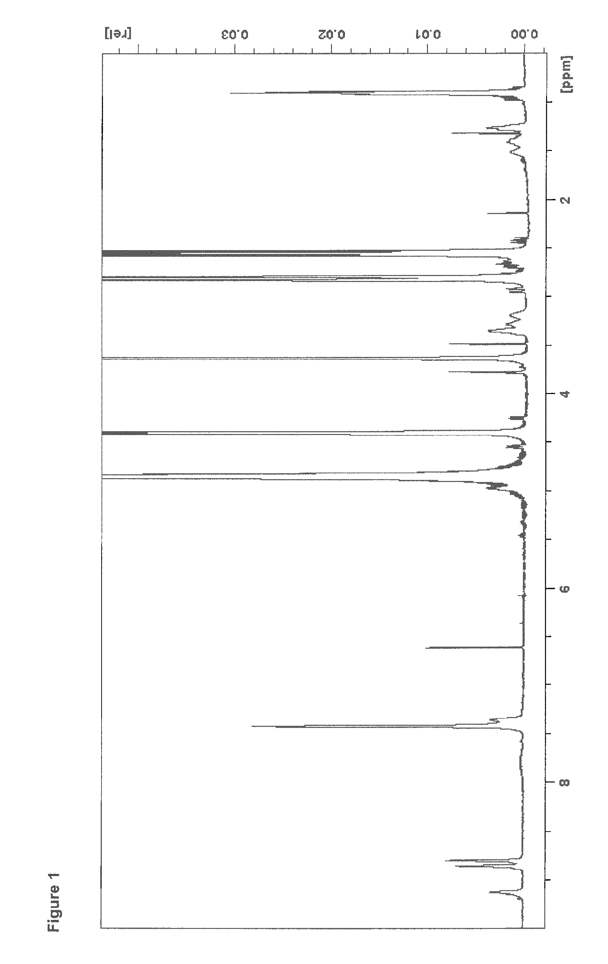

From this solution a sample was taken and investigated by NMR (500 MHz Bruker Avance II ¹HNMR Instrument equipped with Top Spin acquisition software). The corresponding NMR spectrum is included as figure 1. From the NMR it can be seen that the solution contains the malic acid ester of bortezomib in an amount of 52% based on the total amount of bortezomib present (rest is free bortezomib).

### Example 2

### Freeze-dried composition

The solution of example 1 was further processed without isolating the malic acid ester of bortezomib. 5 g of glycine were added to the solution, and it was stirred until a clear solution was obtained. The solution was filtered and cooled to 5°C. The solution was subjected to lyophilization in order to obtain a lyophilized powder. The lyophilized powder was filled in vials to provide vials with 1 mg of bortezomib (free bortezomib and its malic acid ester) and 3.5 mg of bortezomib (as free bortezomib and its malic acid ester). The vials were stored for stability tests.

It should be noted that during example 1 and example 2 all proceedings were carried out under nitrogen gas, and the vials were also filled and sealed under nitrogen gas.

### Example 3

### Preparation of a solution for parenteral administration

A vial prepared in example 2 containing 3.5 mg bortezomib and its malic acid ester was dissolved in 3.5 ml of a 0.9% sodium chloride aqueous solution in order to obtain an intravenous injection of bortezomib. The dissolution of the lyophilized powder is completed in not more than 2 minutes. The reconstituted solution is clear and colorless with a final pH of about 5.

A vial obtained in example 2 containing 3.5 mg bortezomib as malic acid ester was reconstituted with 1 ml of a 0.9% sodium chloride aqueous solution in order to obtain a solution for injection of bortezomib with a concentration of 3.5 mg/ml. Dissolution of the lyophilized powder is completed in not more than 2 minutes. After reconstitution, the solution is clear and colorless with a final pH of about 5.

### Example 4

The vials obtained in example 2 were evaluated as follows.

### Results of Analytical Characterization

**Table 1. Test results of Bortezomib 1 mg and 3.5 mg powder for solution for injection of Example 2**

| **Test sample** | | **Bortezomib PFI** | |
|---|---|---|---|
| Strength | | 1.0 mg | 3.5 mg |

| **Test parameters** | | | |
|---|---|---|---|
| Description | Before reconstitution | White freeze-dried cake | White freeze-dried cake |
| | After reconstitution | Clear and colorless solution | Clear and colorless solution |
| pH after reconstitution | | 4.9 | 5.0 |
| Reconstitution time | | 38 sec | 47 sec |
| Assay of Bortezomib | | 102.8% | 99.3% |
| Related substances | Hydroxy-amide | 0.1% | < 0.1% |
| | Bortezomib amide | < 0.1% | < 0.1% |
| | Any other impurity | < 0.1% | < 0.1% |
| | Total impurities | 0.1% | < 0.1% |
| Water content | | 2.0% | 2.1% |
| Osmolality | | 475 mOsmol/kg | 471 mOsmol/kg |
| Headspace O₂ content | | 0.4% | 0.4% |

### Accelerated stability study

To prove the stability of the product, 1 mg powder for solution for injection drug product was stored at 40°C/75% RH for 2 weeks and 1 month and tested for assay, related substances and water content.

The test results are summarized in **Table 2.**

**Table 2. Test results of Bortezomib 1 mg powder for solution for injection (Example 2) after storage at accelerated conditions (40°C/75% RH)**

| **Test parameters** | | **Results** | | |
|---|---|---|---|---|
| | | **Initial** | **2 weeks** | **1 month** |
| Assay of Bortezomib | | 102.8% | 102.9% | 105.6%¹ |
| Related substances | Hydroxy-amide | 0.1% | < 0.1% | < 0.1% |
| | Bortezomib amide | < 0.1% | < 0.1% | < 0.1% |
| | Any other impurity | < 0.1% | 0.1% | 0.2% |
| | Total impurities | 0.1% | 0.1% | 0.3% |
| Water content² | | 2.0% | 3.5% | 3.9% |

| | | | | |
|---|---|---|---|---|
| ¹ Higher assay result is due to the 4% variability of manual filling. ² Water content increase is due to residual moisture of the stoppers. | | | | |

### In-use stability and compatibility study

To demonstrate the stability of the product upon usage, an in-use stability study was carried out on Bortezomib 3.5 mg powder for solution for injection drug product of Example 2. Both intravenous and subcutaneous administrations were simulated.

The chemical and physical in-use stability of the reconstituted solution has been demonstrated for 8 hours at 25°C stored in the original vial and/or a syringe.

The 3.5 mg powder for solution for injection drug product was reconstituted in either 3.5 ml (for IV administration) or 1.4 ml (for SC administration) of sodium chloride 9 mg/ml and stored either in a vial or in a syringe for eight hours at room temperature under normal indoor lighting. Samples were visually inspected and analyzed for pH, assay and related substances immediately after reconstitution and after storage for 8 hours.

Results of the in-use stability and compatibility study are summarized in **Table 3 and Table 4.**

Immediately after reconstitution and after 8 hours of storage all tested samples had the appearance of clear, colorless solutions and were practically free from visible particles. Reconstitution time was less than 2 minutes; pH of the reconstituted solution was well within the defined range even after 8 hours of storage. No significant differences in the test results for Assay and Related substances were observed upon storage for 8 hours; related substances were below the reporting threshold.

**Table 3. Test results of in-use stability and compatibility study of Bortezomib 3.5 mg powder for solution for injection upon reconstitution for intravenous administration (in 3.5 ml Salsol)**

| **Test parameters** | | **Initial** | **After 8 hours** | |
|---|---|---|---|---|
| | | | **In vial** | **In syringe** |
| Description | Before reconstitution | White freeze-dried cake | N/A | N/A |
| | After reconstitution | Clear, colorless solution, free from visible particles | Clear, colorless solution, free from visible particles | Clear, colorless solution, free from visible particles |
| Reconstitution time | | 69 sec | N/A | N/A |
| pH after reconstitution | | 4.9 | 4.9 | 4.9 |
| Assay of Bortezomib | | 99.9% | 98.7% | 98.7% |
| Related substances | Hydroxy-amide | < 0.1% | < 0.1% | < 0.1% |
| | Bortezomib amide | < 0.1% | < 0.1% | < 0.1% |
| | Any other impurity | < 0.1% | < 0.1% | < 0.1% |
| | Total impurities | < 0.1% | < 0.1% | < 0.1% |

**Table 4. Test results of in-use stability and compatibility study of Bortezomib 3.5 mg powder for solution for injection upon reconstitution for subcutaneous administration (in 1.4 ml Salsol)**

| **Test parameters** | | **Initial** | **After 8 hours** | |
|---|---|---|---|---|
| | | | **In vial** | **In syringe** |
| Description | Before reconstitution | White freeze-dried cake | N/A | N/A |
| | After reconstitution | Clear, colorless solution, free from visible particles | Clear, colorless solution, free from visible particles | Clear, colorless solution, free from visible particles |
| Reconstitution time | | 83 sec | N/A | N/A |
| pH after reconstitution | | 4.9 | 4.9 | 4.9 |
| Assay of Bortezomib | | 97.7% | 98.7% | 98.7% |
| Related substances | Hydroxy-amide | < 0.1% | < 0.1% | < 0.1% |
| | Bortezomib amide | < 0.1% | < 0.1% | < 0.1% |
| | Any other impurity | < 0.1% | < 0.1% < 0.1% | < 0.1% < 0.1% |
| | Total impurities | < 0.1% | < 0.1% | < 0.1% |

### Example 5

Examples 1 to 4 were repeated, except that no glycine was used and the composition was as shown below. The results of the stability tests are also shown below.

| **Components** | **Composition** |
|---|---|
| Bortezomib | 1.0 mg/ml |
| Malic acid | 14.7 mg/ml |
| NaOH | q.s. to adjust pH to 4.9-5.1 |
| WFI | ad 1.0 ml |

| | **Assay** | **Hydroxy amide** | **Bortezomib amide** | **Any other impurity** | **Total impurities** | **Water content** | **Reconstitution time** |
|---|---|---|---|---|---|---|---|
| **Requirement** | **95.0-105.0%** | **NMT 1.0%** | **NMT 1.0%** | **NMT 0.5%** | **NMT 3.0%** | **NMT 4.0%** | **NMT 120 sec** |
| Bulk solution | 101.3% | 0.2% | < 0.1% | < 0.1% | 0.2% | - | - |
| Bulk solution after 1 week at 2-8°C | 101.2% | 0.2% | < 0.1% | < 0.1% | 0.2% | - | - |
| Lyo product | 97.8% | 0.3% | < 0.1% | < 0.1% | 0.3% | 3.0% | 47 sec |
| After 2 weeks at 40°C | 97.7% | 0.2% | < 0.1% | 0.3% | 0.6% | - | - |
| After 1 month at 40°C | 99.9% | 0.2% | < 0.1% | 0.5% | 0.7% | - | - |

### Example 6 (comparative)

The procedure as disclosed in examples 1 and 2 above was followed, except that cysteine was used instead of malic acid and that no glycine was used. The composition is shown below. The NMR data showed that no ester bond was formed between the cysteine and the bortezomib.

In order to improve the stability, an antioxidant was also added to the solution. However, even with the antioxidant sodium metabisulfite, the stability of the freeze-dried product was not satisfactory.

As in example 4, the stability of the lyophilized product was investigated, and the results are shown below:

| **Components** | **Composition** |
|---|---|
| Bortezomib | 1.0 mg/ml |
| L-cysteine | 4.8 mg/ml |
| NaCl | 2.4 mg/ml |
| Sodium metabisulfite | 3.2 mg/ml |
| NaOH | q.s. to pH 5.0 |
| WFI | ad 1.0 ml |

| | | **Assay** | **Hydroxy amide** | **Bortezomib amide** | **Any other impurity** | **Total impurities** |
|---|---|---|---|---|---|---|
| **Requirement** | | **95.0-105.0%** | **NMT 1.0%** | **NMT 1.0%** | **NMT 0.5%** | **NMT 3.0%** |
| | initial bulk | 95.9% | 0.1% | < 0.1% | 0.1% | 0.3% |
| | after 24h at 2-8°C | 95.1% | < 0.1% | < 0.1% | < 0.1% | < 0.1% |
| | 1 lyo product | 91.0% | < 0.1% | 0.1% | 0.6% | 0.7% |
| | after 2 weeks at 40°C | 81.9% | < 0.1% | < 0.1% | 0.6% | 1.0% |
| | after 1 month at 40°C | 76.8% | < 0.1% | 0.2% | 0.6% | 1.1% |

| | | | | | | |
|---|---|---|---|---|---|---|
| ¹Water content: 1.3%, reconstitution time: 129 sec, headspace oxygen: NMT 0.65% | | | | | | |

| **Components** | **Composition** |
|---|---|
| Bortezomib | 1.0 mg/ml |
| L-cystein | 4.8 mg/ml |
| NaCl | 2.4 mg/ml |
| Sodium metabisulfite | 3.2 mg/ml |
| NaOH | q.s. to pH 5.0 |
| Acetonitrile | 30 (V/V) % |
| WFI | ad 1.0 ml |

| | | **Assay** | **Hydroxy amide** | **Bortezomib amide** | **Any other impurity** | **Total impurities** |
|---|---|---|---|---|---|---|
| **Requirement** | | **95.0-105.0%** | **NMT 1.0%** | **NMT 1.0%** | **NMT 0.5%** | **NMT 3.0%** |
| | initial bulk | 108.2% | 0.2% | < 0.1% | 0.2% | 0.5% |
| | after 24h at 2-8°C | 108.0% | 0.3% | < 0.1% | 0.2% | 0.7% |
| | after 24h at 25°C | 107.3% | 0.5% | < 0.1% | 0.5% | 1.4% |
| | 1 lyo product | 90.4% | 0.2% | < 0.1% | 0.2% | 0.4% |

| | | | | | | |
|---|---|---|---|---|---|---|
| 1 Water content: 1.4%, reconstitution time: 1.5 hours. | | | | | | |

### Example 7 (comparative)

Examples 1 and 2 above were repeated, except that citric acid was used instead of malic acid and that no glycine was used. Furthermore, in order to improve the stability an antioxidant (sodium metabisulfite) was added to the composition, which is shown in the table below. NMR data showed that the citric acid ester of bortezomib was formed in the solution. The solution was filtered and cooled to 5°C and then freeze-dried. The stability tests on the freeze-dried powder carried out as described in example 4 above showed that the product was not sufficiently stable.

| **Components** | **Composition** |
|---|---|
| Bortezomib | 1.0 mg/ml |
| Citric acid | 21.0 mg/ml |
| NaCl | 2.4 mg/ml |
| Sodium metabisulfite | 3.2 mg/ml |
| NaOH | q.s. to adjust the pH to 5.0 |
| Acetonitrile | 30 (V/V)% |
| WFI | ad 1.0 ml |

| | **Assay** | **Hydroxy amide** | **Bortezomib amide** | **Any other imp.** | **Total imp.** |
|---|---|---|---|---|---|
| **Requirement** | **95.0-105.0%** | **NMT 1.0%** | **NMT 1.0%** | **NMT 0.5%** | **NMT 3.0%** |
| **With antioxidant** | | | | | |
| Initial bulk solution | 112.2% | 0.6% | < 0.1% | 0.2% | 1.0% |

Despite the presence of an antioxidant and despite the fact that an ester bond formed between citric acid and bortezomib the stability of the lyophilized product was not satisfactory. This is quite surprising, since in the prior art according to WO 2009/154737 the citric acid ester of bortezomib is considered as particularly preferred.

### Example 8

Examples 1 to 4 were repeated but without using acetonitrile in the aqueous solution which is to be freeze-dried.

| **Components** | **Composition of bulk solution** |
|---|---|
| Bortezomib | 1.0 mg/ml |
| Malic acid | 14.7 mg/ml |
| Glycine | 5.0 mg/ml |
| NaOH | q.s. to adjust pH to 4.9-5.1 |
| WFI | ad 1.0 ml |

| | **Assay** | **Hydroxy amide** | **Bortezomib amide** | **Any other imp.** | **Total imp.** |
|---|---|---|---|---|---|
| **Requirement** | **95.0-105.0%** | **NMT 1.0%** | **NMT 1.0%** | **NMT 0.5%** | **NMT 3.0%** |
| Initial bulk solution | 104.3% | 0.1% | < 0.1% | 0.1% | 0.3% |
| After 24 h at 2-8°C | 104.4% | 0.1% | < 0.1% | 0.1% | 0.2% |
| After 24 h at 25°C | 104.0% | 0.1% | < 0.1% | 0.1% | 0.2% |
| After 8 days at 2-8°C | 104.5% | 0.2% | < 0.1% | 0.1% | 0.3% |

| | **Assay** | **Hydroxy amide** | **Bortezomib amide** | **Any other impurity** | **Total impurities** | **Water content** | **Rec. time** |
|---|---|---|---|---|---|---|---|
| **Requirement** | **95.0-105.0%** | **NMT 1.0%** | **NMT 1.0%** | **NMT 0.5%** | **NMT 3.0%** | **NMT 4.0%** | **NMT 120 sec** |
| Bulk solution | 102.1% | 0.1% | < 0.1% | < 0.1% | 0.1% | - | - |
| Lyo product | 100.9% | 0.2% | < 0.1% | 0.1% | 0.2% | 3.1% | 10 sec |
| After 2 weeks at 40°C | 99.7% | 0.2% | < 0.1% | 0.2% | 0.4% | - | - |
| After 1 month at 40°C | 101.4% | 0.2% | < 0.1% | 0.4% | 0.6% | - | - |

It can be seen that even without acetonitrile in the aqueous composition the lyophilized product has an acceptable stability

### Example 9 (comparative)

Example 8 was repeated but using serine instead of malic acid. The compositions and the results of the stability tests are shown below:

| **Components** | **Composition 1** | **Composition 2** |
|---|---|---|
| Bortezomib | 1.0 mg/ml | 1.0 mg/ml |
| L-serine | 12.0 mg/ml | 12.0 mg/ml |
| NaCl | 2.4 mg/ml | 2.4 mg/ml |
| Sodium metabisulphite | 3.2 mg/ml | N/A |
| NaOH | q.s. to adjust the pH to 5.0 | q.s. to adjust the pH to 5.0 |
| WFI | ad 1.0 ml | ad 1.0 ml |

| | **Assay** | **Hydroxy amide** | **Bortezomib amide** | **Any other imp.** | **Total imp.** |
|---|---|---|---|---|---|
| **Requirement** | **95.0-105.0%** | **NMT 1.0%** | **NMT 1.0%** | **NMT 0.5%** | **NMT 3.0%** |
| **With antioxidant** | | | | | |
| initial bulk | 97.5% | 0.3% | < 0.1% | < 0.1% | 0.3% |
| after 24 h at 2-8°C | 95.5% | 0.4% | 0.2% | 0.1% | 0.7% |
| after 24 h at room temp. | 94.2% | 0.6% | 0.4% | 0.3% | 1.5% |

| **Without antioxidant** | | | | | |
|---|---|---|---|---|---|
| initial bulk | 94.4% | 0.6% | < 0.1% | 0.2% | 0.8% |
| after 24 h at 2-8°C | 93.5% | 0.7% | < 0.1% | 0.3% | 1.0% |
| after 24 h at room temp. | 93.8% | 0.8% | < 0.1% | 0.3% | 1.1% |

| | **Assay** | **Hydroxy amide** | **Bortezomib amide** | **Any other impurity** | **Total impurities** | **Water content** |
|---|---|---|---|---|---|---|
| **Requirement** | **95.0-105.0%** | **NMT 1.0%** | **NMT 1.0%** | **NMT 0.5%** | **NMT 3.0%** | **NMT 4.0%** |
| **With antioxidant** | | | | | | |
| bulk | 95.6% | 0.3% | < 0.1% | < 0.1% | 0.3% | N/A |
| lyo product¹ | 76.2% | 10.1% | 0.7% | 1.0% | 12.9% | 7.6% |
| bulk | 99.7% | 1.8% | 0.2% | 0.2% | 2.2% | N/A |
| lyo product² | 78.9% | 13.8% | 0.8% | 1.3% | 18.1% | 7.0% |
| **Without antioxidant** | | | | | | |
| bulk | 91.3% | 1.2% | < 0.1% | 0.5% | 1.7% | N/A |
| lyo product³ | 79.7% | 0.4% | < 0.1% | 0.2% | 0.6% | 4.2% |
| bulk | 94.3% | 0.4% | < 0.1% | 0.2% | 0.6% | N/A |
| lyo product⁴ | 86.9% | 0.4% | < 0.1% | 0.2% | 0.6% | 4.9% |

| | | | | | | |
|---|---|---|---|---|---|---|
| ¹High headspace oxygen content, reconstitution time: 103 sec ² Reconstitution time: 99 sec ³ Reconstitution time: 358 sec ⁴ Reconstitution time: 405 sec | | | | | | |

**Lyo product stability results with serine and without antioxidant**

| | **Assay** | **Hydroxy amide** | **Bortezomib amide** | **Any other imp.** | **Total imp.** |
|---|---|---|---|---|---|
| **Requirement** | **95.0-105.0%** | **NMT 1.0%** | **NMT 1.0%** | **NMT 0.5%** | **NMT 3.0%** |
| **Without antioxidant** | | | | | |
| lyo product, initial | 79.7% | 0.4% | < 0.1% | 0.2% | 0.6% |
| after 2 weeks at 40°C | 81.7% | 0.3% | < 0.1% | 0.2% | 0.5% |

It can be seen that the stability is not satisfactory. In order to improve the stability, the antioxidant sodium metabisulfite was added prior to lyophilization, but even using this antioxidant, the lyophilized product did not have sufficient storage stability.

## Claims

1. An ester compound formed from bortezomib and malic acid or a pharmaceutically acceptable salt of the ester compound.

2. The ester compound or salt thereof according to claim 1, wherein the malic acid is L-malic acid.

3. A pharmaceutical composition comprising the ester compound or salt thereof according to claim 1 or claim 2.

4. The pharmaceutical composition according to claim 3, which is a powder for solution for injection.

5. The pharmaceutical composition according to claim 4, which is a powder obtained by lyophilization.

6. The pharmaceutical composition according to claim 3, which is a solution for injection.

7. A pharmaceutical composition according to any of claims 3 to 6, comprising glycine.

8. The pharmaceutical composition according to claim 7, wherein the mass ratio of bortezomib:glycine is in the range of 1:3 to 1:6, preferably of 1:4 to 1:5.

9. A process for preparing a pharmaceutical composition according to any of claims 3 to 8, comprising contacting bortezomib and malic acid in an aqueous medium or an organic solvent.

10. Process for preparing a pharmaceutical composition according to claim 9, further comprising freeze-drying after the bortezomib and malic acid were contacted in an aqueous medium or an organic solvent to obtain a freeze-dried composition.

11. Process for preparing a pharmaceutical composition according to claim 10, wherein the aqueous composition contains an organic solvent.

12. Process for preparing a pharmaceutical composition according to any of claims 9 to 11, wherein the organic solvent is acetonitrile.

13. Process for preparing a pharmaceutical composition according to any of claims 10 to 12, further comprising reconstitution of the freeze-dried composition of any of claims 10 to 12.

14. The ester compound or salt thereof according to claim 1 or 2 or the pharmaceutical composition according to any of claims 3 to 8 for use in treating cancer.

15. Pharmaceutical composition obtainable by a process which comprises
- mixing bortezomib, malic acid and optionally one or more pharmaceutically acceptable excipients in an aqueous medium or an organic solvent,
- optionally filtering the mixture,
- freeze-drying the mixture and
- optionally reconstituting the freeze-dried mixture in a pharmaceutically acceptable liquid medium.
